# EUROPEAN PATENT SPECIFICATION

(11) **EP 4 413 152 B1**
(45) Date of publication and mention of the grant of the patent: **19.08.2026**
(21) Application number: 22904821.0
(22) Date of filing: 01.12.2022
(51) Int. Cl.: C12Q 1/68

(54) **DIRECT CARBAPENEMASE TRIPLE (KPC-2, OXA-48, NDM-1) PCR DIAGNOSTIC KIT**
DIAGNOSTISCHER KIT FÜR DIREKTE CARBAPENEMASE-TRIPLE (KPC-2, OXA-48, NDM-1)-PCR
KIT DE DIAGNOSTIC DIRECT PAR PCR TRIPLE (KPC-2, OXA-48, NDM-1) DE CARBAPÉNÉMASE

(30) Priority: 04.12.2021 TR 202119090
(43) Date of publication of application: 14.08.2024
(73) Proprietor: Trakya Universitesi Rektorlugu, 22030 Edirne (TR)
(72) Inventor: BUKAVAZ, Sebnem, Edirne (TR)
(74) Representative: Yalçiner Patent and Consulting Ltd.
(86) International application number: PCT/TR2022/051394
(87) International publication number: WO 2023/107058

(56) References cited:
- WO-A1-2020/072858
- WO-A1-2020/257691
- CN-A- 109 762 915
- US-A1- 2020 030 366
- AVERSHINA EKATERINA, SHAPOVALOVA VALERIA, SHIPULIN GERMAN: "Fighting Antibiotic Resistance in Hospital-Acquired Infections: Current State and Emerging Technologies in Disease Prevention, Diagnostics and Therapy", FRONTIERS IN MICROBIOLOGY, vol. 12, XP093083814, DOI: 10.3389/fmicb.2021.707330
- BUKAVAZ, S. ET AL.: "blaKPC-2 and blaOXA-48 producing Klebsiella pneumoniae found in a Turkish hospital in the Balkans", AFRICAN JOURNAL OF MICROBIOLOGY RESEARCH, vol. 12, no. 16, 28 April 2018 (2018-04-28), pages 370 - 379, XP055746407, DOI: 10.5897/AJMR2018.8841

## Description

### Technical Field of Invention

The present invention relates to a PCR-based rapid diagnostic kit for the detection of carbapenemases causing antibiotic (carbapenem) resistance.

### State of the Art Related to the Invention (Background)

Carbapenemases are β-lactamases that hydrolyze penicillin, that hydrolyze cephalosporins in most cases and hydrolyze carbapenems and monobactams to a certain extent.

Infections caused by extended-spectrum beta-lactamases (ESBLs) and carbapenemase-producing Gram-negative bacteria in recent years have posed serious therapeutic problems, especially in hospitalized patients and those with underlying immunosuppressive conditions.

The most commonly detected carbapenemases in the Enterobacteriaceae family worldwide are Klebsiella pneumoniae, *Acinetobacter baumannii* carbapenemase (KPC), New Delhi metallo-β-lactamase (NDM), and oxacillinase-48 OXA-48; *E. coli* carbapenemases KPC, OXA, NDM, VIM and IMP; Enterococcus spp. common Vancomycin resistance genes Van A, VanB, and *Staphylococcus aureus* Methicillin resistance gene MecA enzymes.

The carbapenems are the antibiotics with the broadest spectrum in the beta-lactam class with rapid bactericidal action and are widely used in infections caused by many aerobic and anaerobic microorganisms with their broad antibacterial spectrum.

In the state of the art; Carbapenemases are identified in routine microbiology laboratories by traditional culture and antibiogram determination.

Using the traditional method of bacterial identification and antibiogram used in routine microbiology laboratories, the bacteria are first cultured from the patient and after 24 hours of incubation, the bacteria are named using automated systems. The working principle of the systems is based on the metabolites used by the bacteria during reproduction and the resulting enzymes and pH changes are basically named based on their biochemical properties. Although there is no technical problem in the use of these methods for the said Enterobacteriaceae member bacteria, at least 48 hours are needed for detection and during this time, resistance enzymes spread rapidly from one patient to another.

As a secondary procedure, the antibiogram uses automated systems to determine which antibiotic the said bacteria is susceptible to. During the said procedure, while the presence of the resistance enzyme is indicated, no definitive judgment can be made about the details and type of resistance. Thus, it is not possible to prevent infectiousness and thus outbreaks by identifying the type of plasmid-mediated enzyme that is dangerous and spreads rapidly among patients and isolating a patient from other patients.

Phenotypic Carba NP and the Modified Hodge Test (MHT) are also used for the identification of carbapenemases in the known state of the art. Although MHT is highly susceptible in *Klebsiella pneumoniae,* it does not perform well in other Enterobacteriaceae members. Furthermore, in the state of the art, these tests confirm the presence of a general carbapenemase but cannot identify the precise name of the enzyme causing resistance. Although the Carba NP test is reliable, it is not preferred due to its excessive cost.

Usually, conventional PCR or internationally produced kits are used for the determination of enzymes in our country. The cost of these kits is quite high as they contain many enzymes other than the important (Kpc-2, Oxa-48, Ndm-1, IMP, VIM, VanA, VanB, mecA, mcr-1, BIC) enzymes that are frequently seen in our country and cause problems and are produced abroad. The determination of resistance also takes a longer time.

The patent numbered US 9,914,980 B2 relates to a kit for the identification of carbapenemase genes, which determines more than 170 carbapenemase enzyme types. Therefore, resistance determination takes a considerably long time.

US20200030366A1 discloses a method of treating or preventing a bacterial infection, or decreasing antibiotic resistance of gastrointestinal microbiome, in a critically ill patient or an immunosuppressed patient, comprising administering an inulin to the patient.

The bacterial drug resistance gene detection method provided by CN109762915A includes the following steps: extracting the genomic DNA of the bacteria to be tested, and amplifying the genomic DNA by using a complete set of single-stranded DNA to obtain an amplified product; based on the Ampliseq technology, using the amplified product to construct a sequencing library; sequencing the sequencing library to obtain a sequencing result, and comparing the sequencing result with the drug resistance gene sequence to determine whether the bacteria to be tested carry the drug resistance gene.

WO2020257691A1 discloses a method that detects pathogens in a biological sample by using a multiplexed amplification reaction to simultaneously amplify a broad panel of pathogen target nucleic acids, including genus-level targets, Gram-positive and Gram-negative bacterial targets, and resistance gene targets. The amplified targets are then detected to determine pathogen presence, with the method achieving at least 90% coverage of pathogen species associated with infections of the sample.

WO2020257691A1 a method for detecting antibiotic resistance genes in a biological sample. The method uses a multiplex nucleic acid amplification reaction to simultaneously amplify target sequences from two or more antibiotic resistance genes, selected from a specified list, with the requirement that at least one gene comes from a defined subset. After amplification, the method detects the amplified nucleic acids to determine whether the corresponding resistance genes are present in the sample. The method is highly sensitive, capable of individually detecting resistance genes from pathogens present at concentrations as low as 10 cells per mL or less of the biological sample.

### Brief Description and Objects of the Invention

The present invention aims to design a PCR-based rapid diagnostic kit for the detection of antibiotic-resistant carbapenemases.

The PCR-based rapid diagnostic kit of the invention enables rapid (2-3 hours) identification of enzymes that cause resistance to antibiotics and detects which enzyme causes resistance. In the case of a plasmid-mediated enzyme, the patient is rapidly isolated, and the spread of resistance is prevented.

The rapid spread of resistance enzymes from one patient to another, which is important for patients with high comorbidity hospitalized in intensive care units, is prevented and the patient is isolated from other patients, and infectiousness is prevented.

The contents of the kit of the invention can be simply designed to correspond to the intended use. For example, kits to be used for diagnostic purposes in microbiology routine laboratories can be produced based on probe hybridization technique, while those to be used in screening tests and epidemiological research studies can be produced with Conventional Single or Multiplex Sybr-Green, Probe-Hybridization techniques.

The kit of the invention enables rapid diagnosis (2-3 hours) of important (Kpc-2, Oxa-48-23-24-58, Ndm-1, IMP, VIM, VanA, VanB, mecA, mcr-1, BIC) resistance genes, which are frequently encountered in our country and which pose a danger since their spread is plasmid-mediated, and has a very low cost.

### Descriptions of the Figures Illustrating the Invention

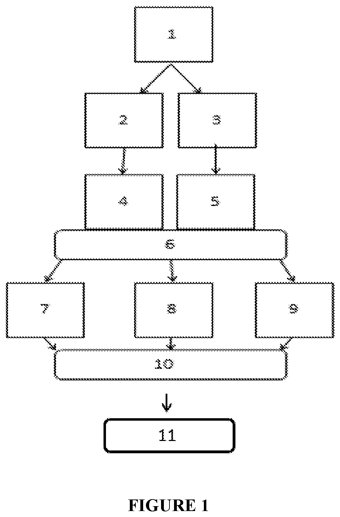

**Figure 1****:** Workflow diagram

### Descriptions of the elements/sections/parts that constitute the invention

**1.** Bacteria reference strains such as carbapenemase-positive *Escherichia coli, Klebsiella pneumoniae, Acinetobacter spp., Pseudomonas aeruginosa, Enterococcus faecalis, Enterococcus faecium, Staphylococcus aureus,* etc.
**2.** Clinical sample
**3.** Bacterial culture from a clinical sample
**4.** Direct DNA isolation from a clinical sample
**5.** DNA isolation from bacterial culture
**6.** Standardization of DNA isolation, determination of specificity and susceptibility
**7.** Conventional PCR (blaKPC-2, blaOXA-48, blaNDM-1, *bla*OXA-23, *bla*OXA-24, *bla*OXA-58, blaOXA-204, blaOXA- 181, *bla*VIM*, bla*IMP, *bla*BIC*,* mecA, vanA, vanB)
**8.** Multiplex PCR
**9.** Quantitative Sybr-Green Multiplex PCR
**10.** Quantitative Prob-Hibridizasyon
**11.** DNA sequence and variant analysis

### Detailed Description of the Invention

The PCR-based diagnostic kit of the invention for the detection of antibiotic-resistant carbapenemases comprises; DNA primer pairs NO:1, NO:2 and NO:3, NO:4, NO:5, NO:6, NO:7, NO:8, NO:9, NO:10, NO:11, NO:12, NO:13, NO:14, NO:15, NO:16, NO:17, NO:18, NO:19, NO:20, NO:21, NO:22, NO:23, NO:24, NO:25, NO:26, NO:27, NO:28, NO:29, NO:30 to detect enzyme types from the carbapenemase group, including *Klebsiella pneumoniae* carbapenemase (KPC) belonging to the carbapenemase group, New Delhi metallo-β-lactamase (NDM), oxacillinase-48 (Oxa-48), Colistin Resistance (MCR), *Oxacillinase-23* (Oxa-23), *Oxacillinase-24* (Oxa-24), *Oxacillinase-58* (Oxa-58), *Pseudomonas aeruginosa* carbapenemases (*bla*VIM*, bla*IMP, and *bla*BIC)*,* enterococcal Vancomycin resistance genes (vanA, vanB), *Staphylococcus aureus* methicillin resistance gene (MecA).

According to the process step sequence shown in Figure 1; the invention is firstly produced by accumulating bacteria (1) such as Carbapenemase-positive *Escherichia coli, Klebsiella pneumoniae, Acinetobacter spp., Pseudomonas aeruginosa, Enterococcus fecalis, E. Faecium and Staphylococcus aures.* Afterward, clinical samples (2) are prepared in routine diagnostic laboratories as recommended in the selected DNA isolation kit and bacterial culture (3) is produced from clinical samples such as blood, endotracheal aspirate, urine, and sputum sent to the laboratory. Direct DNA isolation from clinical samples (4) and DNA isolation from bacterial culture (5) are performed simultaneously with a DNA isolation kit or boiling method. If the PCR step is not to be performed immediately, the samples are stored at +4°C for a short time (a few weeks) and at -80°C for a period of 6 months. After standardization of DNA isolation, determination of specificity and susceptibility (6), kits that enable the detection of the appropriate number of enzymes for the study objects can be produced by Conventional Single (7) or Multiplex (8) and Multiplex Sybr-Green (9), Probe-Hybridization (10) and measurements can be performed. While single conventional PCR can be performed with any of *bla*KPC-2, *bla*OXA-48, *blaNDM-1, bla*OXA-23, *bla*OXA-24, *bla*OXA-58, blaOXA-204, blaOXA- 181, *bla*VIM*, bla*IMP*, bla*BIC*,* mecA, vanA, vanB *bla*MCR-1., multiplex PCR can be performed with any two or three of the *bla*KPC-2, *bla*OXA-48, *bla*NDM-1, *bla*OXA-23, *bla*OXA-24, *bla*OXA-58, blaOXA-204, *bla*OXA- 181, *bla*VIM*, bla*IMP, *bla*BIC*,* mecA, vanA, vanB *bla*MCR-1 gene regions or in a single reaction depending on the number of PCR device channels, and probe hybridization can be performed with single and/or multiple enzymes. In this art, by using primers targeting the DNA regions encoding the problematic resistance enzymes, PCR kits are produced that enable the amplification of the selected DNA regions ready to be designed in different types suitable for the objects of the study. Table 1 shows the DNA primer pairs included in the kit that are specific for the detection of different carbapenemase genes.

**Table 1: DNA primer pairs included in the kit that are specific for the detection of different carbapenemase genes**

| | Forward primer | Reverse primer |
|---|---|---|
| **NDM-1** | TGGATCAAGCAGGAGATCAAC (NO: 1) | ATCAGGCAGCCACCAAAAG (NO: 2) |
| **KPC-2** | ACCATTCGCTAAACTCGAAC (NO: 3) | CAATCAACAAACTGCTGCC (NO: 4) |
| **OXA-48** | AGCCGCAGACAATTAGCCAC (NO: 5) | CGCCAAGCCATCACAAAAGAAG (NO: 6) |
| **MCR-1** | CTCCAAAATGCCCTACAGAC (NO:7) | ATAATGACTGCTGAACGCC (NO:8) |
| **OXA-23** | AATACAGAATATGTGCCAGCC (NO:9) | TTGCCCAACCAGTCTTTCC (NO: 10) |
| **OXA-24** | GCCTAGAGCTAATGCAGAAAG (NO:11) | TAACACCCATTCCCCATCC (NO: 12) |
| **OXA-58** | GCATTTAGACCGAGCAAAAAC (NO:13) | CTCTGCGCTCTACATACAAC (NO:14) |
| ***Klebsiella pneumoniae*** | CCACACTGGAACTGAGACAC (NO:15) | TCACATCCGACTTGACAGAC (NO:16) |
| **16S-rRNA Internal Control KPIC** | | |
| ***Acinetobacter baumannii*** | GGGAGCAAACAGGATTAGATAC (NO:17) | ACCCAACATCTCACGACAC (NO:18) |
| **16S-rRNA** | | |
| **IMP** | TCATTTTCATAGCGACAGCAC (No: 19) | TCCTTTCAGGCAGCCAAAC (No: 20) |
| **VIM** | CGGAGAGGACAAAGCAAGAC (No: 21) | ATTCAATCCACCCTACCCAC (No: 22) |
| **BIC** | TTTTGACTTTCGAGCGGACG SEQ ID No: 23 | GAAGCAAATTTGTCGCACCG SEQ ID No:24 |
| **MecA** | TGGCTATCGTGTCACAATCG SEQ ID No:25 | ACGTTGTAACCACCCCAAGA SEQ ID No:26 |
| **VanA** | TTTTGACTTTCGAGCGGACG SEQ ID No:27 | GAAGCAAATTTGTCGCACCG SEQ ID No:28 |
| **VanB** | GGATAGCTACTCCCGCCTTT SEQ ID No:29 | CTAGACCTCTACAGCCGAGC SEQ ID No:30 |

The content of these kits contains a solution and materials called master mix, which includes Taq DNA polymerase enzyme and primers specific to the region to be amplified, as well as tubes prepared as control. With the PCR kit selected according to the suspected resistance enzyme, the reaction is completed in a short time (approximately 3-4 hours) in a thermal cycling device. For the final step, DNA sequence and variant analysis (10), PCR samples are run on a 1% agarose gel at 100V for 30 minutes if the traditional PCR kit is selected. If a Real-Time PCR kit is selected, Melt-Curve analysis and accurate melting points as well as internal amplification controls (IAK) are evaluated, and results are recorded. The enzyme variation will also be statistically evaluated by examining the nucleotide differences according to the DNA Sequence Analysis result of the selected samples.

## Claims

1. PCR-based diagnostic kit for detection of antibiotic-resistant carbapenemase, **characterized in that** it comprises DNA primer pairs NO:1, NO:2 and NO:3, NO:4, NO:5, NO:6, NO:7, NO:8, NO:9, NO:10, NO:11, NO:12, NO:13, NO:14, NO:15, NO:16, NO:17, NO:18, NO:19, NO:20, NO:21, NO:22, NO:23, NO:24, NO:25, NO:26, NO:27, NO:28, NO:29, NO:30 including *Klebsiella pneumoniae* carbapenemase (KPC) belonging to the carbapenemase group, New Delhi metallo-β-lactamase (NDM), oxacillinase-48 (Oxa-48), Colistin Resistance (MCR), *Oxacillinase-23* (Oxa-23), *Oxacillinase-24* (Oxa-*24), Oxacillinase-58* (Oxa-58), *Pseudomonas aeruginosa* carbapenemases (*bla*VIM*, bla*IMP*,* and *bla*BIC)*,* enterococcal Vancomycin resistance genes (vanA, vanB), *Staphylococcus aureus* methicillin resistance gene (MecA) to detect enzyme types from the carbapenemase group, and as Internal Controls *Klebsiella pneumoniae* 16S-rRNA Internal Control KPIC and *Acinetobacter baumannii* 16S-rRNA (ABIC).

2. A method of operation of the PCR-based diagnostic kit according to claim 1, **characterized in that** it comprises the following process steps;
• producing bacteria (1) such as carbapenemase-positive *Escherichia coli* and *Klebsiella pneumoniae, Pseudomonas aeruginosa, Enterococcus faecium, Enterococcus fecalis, Staphylococcus aureus,*
• obtaining bacterial cultures (3) from clinical samples (2), such as blood, endotracheal aspirate, or sputum,
• direct DNA isolation from clinical specimens (4) and DNA isolation from bacterial culture (5),
• standardization of DNA isolation, determination of specificity and susceptibility (6),
• producing kits for enzyme detection by Conventional Single (7) or Multiplex (8) and Multiplex Sybr-Green (9), Probe-Hybridization (10) techniques,
• DNA sequence and variant analysis (11).

## Patentansprüche

1. PCR-basiertes Diagnostik- Kit zum Nachweis antibiotikaresistenter Carbapenemasen , **dadurch gekennzeichnet, dass** es DNA-Primerpaare Nr. 1, Nr.2 und Nr. 3, Nr.4, Nr.5, Nr.6, Nr.7, Nr.8, Nr.9, Nr.10, Nr.11, Nr.12, Nr.13, Nr.14, Nr.15, Nr.16, Nr.17, Nr.18, Nr.19, Nr.20, Nr.21, Nr. 22, Nr.23, Nr.24, Nr.25, Nr.26, Nr.27, Nr.28, Nr.29, Nr.30 umfasst, darunter *Klebsiella pneumoniae* Carbapenemase (KPC), die zur Carbapenemase-Gruppe gehört, New Delhi Metallo- β-Lactamase (NDM), Oxacillinase-48 (Oxa-48), Colistin-Resistenz (MCR), *Oxacillinase-23* (Oxa-23), *Oxacillinase-24* (Oxa -24), *Oxacillinase-58* (Oxa-58), Carbapenemasen von *Pseudomonas aeruginosa (bla*VIM *, bla*IMP *und* blaBIC), Vancomycin-Resistenzgene von Enterokokken (vanA, vanB), Methicillin-Resistenzgen *von Staphylococcus aureus* (MecA) zum Nachweis von Enzymtypen aus der Carbapenemase-Gruppe sowie als interne Kontrollen *Klebsiella pneumoniae* 16S-rRNA Internal Control KPIC und *Acinetobacter baumannii* 16S-rRNA (ABIC).

2. Verfahren zur Durchführung des PCR-basierten Diagnostik-Kits nach Anspruch 1, **dadurch gekennzeichnet, dass** es die folgenden Verfahrensschritte umfasst;
• produzierende Bakterien (1) wie beispielsweise Carbapenemase-positive *Escherichia coli* und *Klebsiella pneumoniae, Pseudomonas aeruginosa, Enterococcus faecium, Enterococcus faecalis, Staphylococcus aureus,*
• gewinnung von Bakterienkulturen (3) aus klinischen Proben (2), wie Blut, endotrachealem Aspirat oder Sputum,
• direkte DNA-Isolierung aus klinischen Probenmatrices (4) und DNA-Isolierung aus Bakterienkulturen (5),
• standardisierung der DNA-Isolierung, Bestimmung der Spezifität und Suszeptibilität (6),
• herstellung von Kits für den Enzymnachweis durch konventionelle Single (7) oder Multiplex (8) und Multiplex Sybr-Green (9), Sonden-Hybridisierung (10) -Techniken,
• DNA-Sequenz- und Variantenanalyse (11).

## Revendications

1. Kit de diagnostic basé sur la PCR pour la détection de carbapénémases résistantes aux antibiotiques, **caractérisé en ce qu'**il comprend des paires d'amorces ADN NO: 1, NO: 2 et NO: 3, NO: 4, NO: 5, NO: 6, NO: 7, NO: 8, NO: 9, NO: 10, NO: 11, NO: 12, NO: 13, NO: 14, NO: 15, NO: 16, NO: 17, NO: 18, NO: 19, NO: 20, NO: 21, NO: 22, NO: 23, NO: 24, NO: 25, NO: 26, NO: 27, NO: 28, NO: 29, NO: 30 y compris *Klebsiella pneumoniae* carbapenemase (KPC) appartenant au groupe des carbapénémases, la métallo-β-lactamase de New Delhi (NDM), *l'oxacillinase-*48 (Oxa-48), la résistance à la colistine (MCR), *l'oxacillinase-*23 (Oxa-23), *l'oxacillinase-*24 (Oxa-24), *l'oxacillinase-*58 (Oxa-58), les carbapénémases de *Pseudomonas aeruginosa* (*bla*VIM, *bla*IMP et *bla*BIC)*,* les gènes de résistance à la vancomycine des entérocoques (vanA, vanB), le gène de résistance à la méthicilline de *Staphylococcus aureus* (MecA) pour détecter les types d'enzymes du groupe des carbapénémases, et comme contrôles internes, le contrôle interne 16S-rRNA de *Klebsiella pneumoniae* (KPIC) et le 16S-rRNA *d'Acinetobacter baumannii* (ABIC).

2. Procédé de fonctionnement du kit de diagnostic basé sur la PCR selon la revendication 1, **caractérisé en ce qu'**il comprend les étapes suivantes :
• production des bactéries (1) telles que *Escherichia coli* et *Klebsiella pneumoniae, Pseudomonas aeruginosa, Enterococcus faecium, Enterococcus faecalis, Staphylococcus aureus* carbapénémases positives,
• obtention des cultures bactériennes à partir d'échantillons cliniques (2), tels que du sang, de l'aspirat endotrachéal ou des expectorations (3),
• l'isolation directe de l'ADN à partir des spécimens cliniques (4) et l'isolation de l'ADN à partir de cultures bactériennes (5),
• normalisation de l'isolation de l'ADN, détermination de la spécificité et de la sensibilité (6),
• production de kits pour la détection d'enzymes par les techniques conventionnelles simples (7) ou multiplex (8) et multiplex Sybr-Green (9), les techniques d'hybridation de sondes (10),
• analyse des séquences d'ADN et des variants (11).
